(19) Europäisches Patentamt
European Patent Office
Office européen des brevets

(11) Veröffentlichungsnummer : **0 050 780**
**B1**

(12) # EUROPÄISCHE PATENTSCHRIFT

(45) Veröffentlichungstag der Patentschrift : **28.12.83**

(21) Anmeldenummer : **81108195.9**

(22) Anmeldetag : **12.10.81**

(51) Int. Cl.³ : **C 07 D263/58**, C 07 D263/60, C 07 D263/62, C 08 G 59/40, C 08 G 18/78, C 08 G 73/00

(54) **Isocyanato-oxazolinone, ein Verfahren zur Herstellung von Oxazolin-2-on-Ringe aufweisenden Kunststoffvorläufern und deren Verwendung zur Herstellung von hochmolekularen Kunststoffen.**

(30) Priorität : **23.10.80 DE 3039929**

(43) Veröffentlichungstag der Anmeldung : **05.05.82 Patentblatt 82/18**

(45) Bekanntmachung des Hinweises auf die Patenterteilung : **28.12.83 Patentblatt 83/52**

(84) Benannte Vertragsstaaten : **BE DE FR GB IT**

(56) Entgegenhaltungen :
**DD-A- 105 230**
**DE-A- 2 429 562**
**DE-B- 1 595 417**
**C.A. 88(1978) Nr. 38384h**

(73) Patentinhaber : **BAYER AG**
**Zentralbereich Patente, Marken und Lizenzen**
**D-5090 Leverkusen 1, Bayerwerk (DE)**

(72) Erfinder : **Rasshofer, Werner, Dr.**
**Wolfskaul 10**
**D-5000 Köln 80 (DE)**
Erfinder : **Grögler, Gerhard, Dr.**
**von-Diergardt-Strasse 46**
**D-5090 Leverkusen (DE)**
Erfinder : **Meyborg, Holger, Dr.**
**Bergisch-Gladbacher-Strasse 1**
**D-5068 Odenthal (DE)**

**EP 0 050 780 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Isocyanato-oxazolinone, ein Verfahren zur Herstellung von Oxazolin-2-on-Ringe aufweisenden Kunststoffvorläufern und deren Verwendung zur Herstellung von hochmolekularen Kunststoffen

Die vorliegende Erfindung betrifft aromatische Isocyanato-oxazolin-2-one, d. h. aromatische Isocyanate mit ankondensiertem Oxazolin-2-on-Ring, welche Verbindungen darstellen, die unterschiedliche Reaktivzentren mit einer unterschiedlichen Reaktionsbereitschaft gegenüber zu Additions- oder Kondensationsreaktionen befähigten Verbindungen aufweisen. Die Erfindung betrifft außerdem ein Verfahren zur Herstellung von Oxazolin-2-on-Ringe aufweisenden Kunststoffvorläufern, bei welchem man sich dieser unterschiedlichen Reaktionsbereitschaft der Reaktivgruppen der erfindungsgemäßen Verbindungen bedient, indem man diese im Sinne einer Isocyanat-Additionsreaktion mit gegenüber Isocyanatgruppen reaktionsfähige Gruppen aufweisenden Kunststoffvorläufern zur Reaktion bringt, sowie die Verwendung der so erhaltenen Verfahrensprodukte zur Herstellung von hochmolekularen Kunststoffen.

Die nachstehend näher beschriebenen erfindungsgemäßen Isocyanato-oxazolin-2-one können als aromatische Polyisocyanate betrachtet werden, deren Isocyanatgruppen teilweise (in Form des Oxazolin-2-on-Ringes) blockiert sind. Bei der Umsetzung dieser « teilblockierten » Polyisocyanate mit Verbindungen mit gegenüber Isocyanatgruppen reaktionsfähigen Gruppen entstehen demzufolge Kunststoffvorläufer mit in Form von Oxazolin-2-on-Ringen blockierten Isocyanatgruppen. Demzufolge eignen sich die nachstehend näher beschriebenen Verfahrensprodukte für alle Einsatzgebiete von blockierten Polyisocyanaten, wobei jedoch bei dieser, nachstehend näher beschriebenen, Verwendung der erfindungsgemäßen Verfahrensprodukte im Gegensatz zu den klassischen blockierten Polyisocyanaten eine Abspaltung von Blockierungsmitteln unterbleibt. Wegen des in den Oxazolinon-Ringen vorliegenden reaktionsfähigen Wasserstoffatoms sind die nachstehend näher beschriebenen erfindungsgemäßen Verfahrensprodukte jedoch auch geeignete Reaktionspartner für zu Additionsreaktionen befähigte Verbindungen, wie z. B. Polyisocyanate oder Polyepoxide. Bei der nachstehend näher beschriebenen erfindungsgemäßen Verwendung der erfindungsgemäßen Verfahrensprodukte kann man sich somit der unterschiedlichsten Methoden bedienen, um die Oxazolin-2-on-Ringe aufweisenden Kunststoffvorläufer in hochmolekulare Kunststoffe zu überführen.

Gegenstand der vorliegenden Erfindung sind somit Isocyanato-oxazolin-2-one der Formel

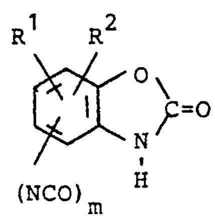

in welcher

$R^1$ und $R^2$ entweder für gleiche oder verschiedene Reste stehen und Wasserstoff, einen aliphatischen Kohlenwasserstoffrest mit 1 bis 4 Kohlenstoffatomen oder Chlor bedeuten oder gemeinsam für einen ankondensierten, gegebenenfalls mit einer Isocyanatgruppe substituierten Benzolring stehen oder wobei

$R^1$ die genannte Bedeutung hat und $R^2$ für einen Rest der Formel

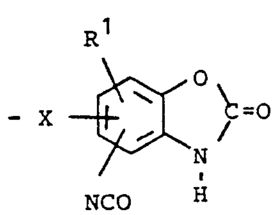

steht, wobei

X für eine Alkylen-Brücke steht und

m für 1 steht bzw. im Falle des Vorliegens eines Isocyanato-substituierten, ankondensierten aromatischen Rings auch für 0 stehen kann.

Gegenstand der vorliegenden Erfindung ist auch ein Verfahren zur Herstellung von unter dem Einfluß von Hitze und gegebenenfalls in Gegenwart von Kettenverlängerungs- bzw. Vernetzungsmitteln zu hochmolekularen, gegebenenfalls vernetzten Kunststoffen ausreagierenden, Oxazolin-2-on-Ringe aufweisenden Kunststoffvorläufern, dadurch gekennzeichnet, daß man mindestens zwei Hydroxylgruppen und/oder Aminogruppen aufweisende Verbindungen der als Reaktionspartner für organische Polyisocyanate aus der Polyurethanchemie bekannten Art mit Isocyanato-oxazolin-2-onen der obengenannten allgemeinen Formel im Sinne einer Additionsreaktion, gegebenenfalls unter Mitverwendung von

Oxazolin-2-on-freien Polyisocyanaten der aus der Polyurethanchemie bekannten Art ein- oder zweistufig, umsetzt, wobei man die Reaktionspartner in (i) einem Äquivalentverhältnis von Isocyanatgruppen des Isocyanatoxazolin-2-ons zu gegenüber Isocyanatgruppen reaktionsfähigen Gruppen des Kunststoffvorläufers von 3 : 1 bis 1 : 3 und (ii) einem Äquivalentverhältnis von Isocyanatgruppen der Oxazolin-2-on-freien Polyisocyanate in gemäß (i) gegebenenfalls vorliegenden überschüssigen, gegenüber Isocyanatgruppen reaktionsfähigen Gruppen von 0 : 1 bis 3 : 1 entsprechenden Mengen einsetzt.

Gegenstand der vorliegenden Erfindung ist schließlich auch die Verwendung der erfindungsgemäßen Verfahrensprodukte zur Herstellung von hochmolekularen Kunststoffen, dadurch gekennzeichnet, daß man diese Kunststoffvorläufer, gegebenenfalls in Gegenwart von Verbindungen, die mindestens 2, mit Oxazolin-2-on-Ringen im Sinne einer Additionsreaktion reaktionsfähigen Gruppen enthalten, ausgewählt aus der Gruppe bestehend aus organischen Polyisocyanaten, organischen Polyaminen mit primären und/oder sekundären Aminogruppen und organischen Epoxiden, einer Hitzebehandlung im Bereich von 100 bis 200 °C unterwirft.

Die Herstellung der erfindungsgemäßen Isocyanato-oxazolin-2-one kann beispielsweise durch Phosgenierung der ihnen zugrundeliegenden Amine der Formel

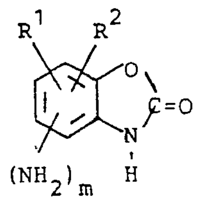

erfolgen, in welcher

$R^1$ und $R^2$ für gleiche oder verschiedene Reste stehen und Wasserstoff, einen aliphatischen Kohlenwasserstoffrest mit 1 bis 4 Kohlenstoffatomen oder Chlor bedeuten oder gemeinsam für einen ankondensierten, gegebenenfalls mit einer Aminogruppe $-NH_2$ substituierten Benzolring stehen oder wobei $R^1$ die genannte Bedeutung hat und $R^2$ für einen Rest der Formel

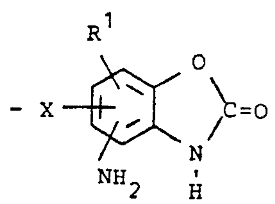

steht, wobei

X die obengenannte Bedeutung hat und

m für 1 steht bzw. im Falle des Vorliegens eines aminosubstituierten, ankondensierten aromatischen Rings auch für 0 stehen kann.

Bevorzugt werden solche Verbindungen der genannten allgemeinen Formel eingesetzt, für welche $R^1$ und $R^2$ für gleiche oder verschiedene Reste stehen und Wasserstoff, einen aliphatischen Kohlenwasserstoffrest mit 1 bis 4 Kohlenstoffatomen oder Chlor, insbesondere jedoch Wasserstoff bedeuten und m für die Zahl 1 steht.

Bei den entsprechenden Phosgenierungsprodukten handelt es sich um die bevorzugten erfindungsgemäßen Verbindungen.

Die Herstellung von als Ausgangsmaterial geeigneten aromatischen Aminen der genannten allgemeinen Formel, für welche $R^1$ und $R^2$ für Wasserstoff, einen $C_1$-$C_4$-Alkylrest oder Chlor stehen, kann beispielsweise nach der von J. Sam und J.L. Valentine in J. Pharm. Sci. *58*, 1 043 ff. (1969) beschriebenen Methode erfolgen. Die Herstellung der Amine der genannten allgemeinen Formel, für welche $R^1$ und $R^2$ für einen ankondensierten, gegebenenfalls aminosubstituierten Benzolring stehen, erfolgt beispielsweise dergestalt, daß man o-Aminonaphthole mit Phosgen oder Harnstoff in ein einen ankondensierten Oxazolin-2-on-Ring aufweisendes Naphthalin überführt, welches durch Nitrierung und anschließende Reduzierung der Nitrogruppe in die entsprechende Aminoverbindung überführt werden kann. Die Herstellung von Aminen der genannten allgemeinen Formel, für welche $R^2$ für einen Rest der Formel

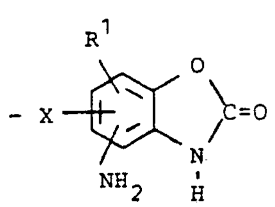

steht, kann beispielsweise nach folgendem Formelschema durch an sich bekannte Umsetzungen erfolgen :

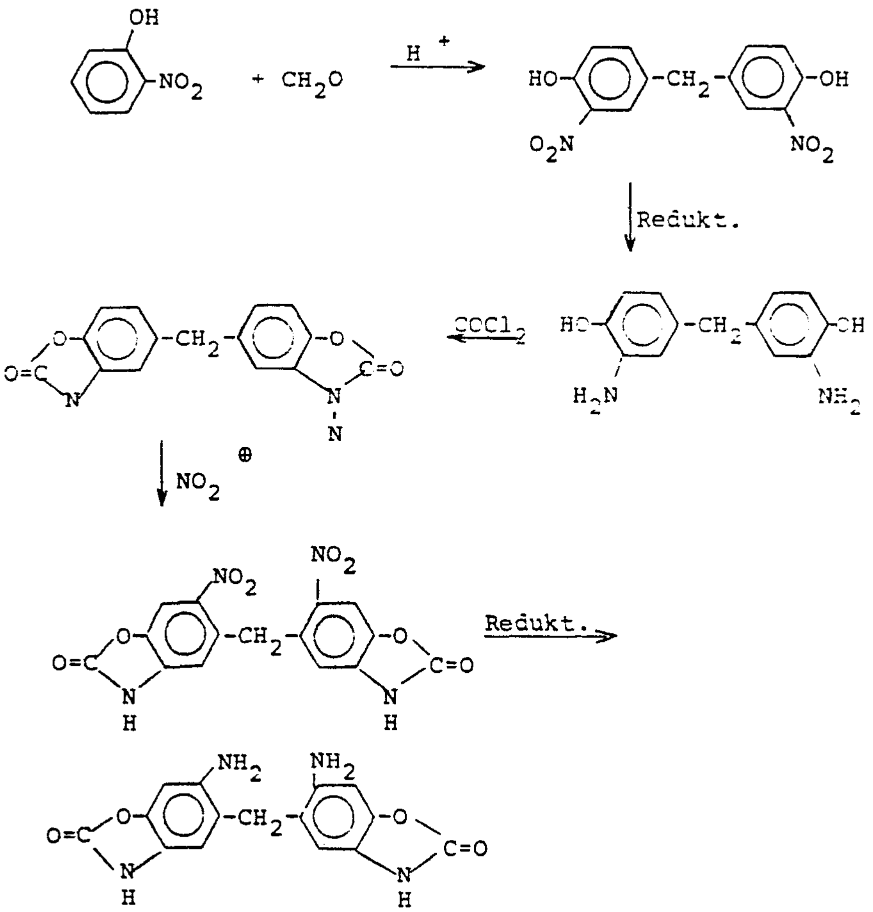


Typische Beispiele aromatischer Amine mit ankondensiertem Oxazolin-2-on-Ring sind 4-Aminobenzoxazolin-2-on, 5-Aminobenzoxazolin-2-on, 6-Aminobenzoxazolin-2-on, 7-Aminobenzoxazolin-2-on, 4-Amino-6-methylbenzoxazolin-2-on, 5-Amino-6-methyl-benzoxazolin-2-on, 5-Amino-6-chlorbenzoxazolin-2-on, 9-Aminobenzo(g)benzoxazolin-2-on, 5-Aminobenzo(g)benzoxazolin-2-on, 4-Aminobenzo(e)-benzoxazolin-2-on, 8-Aminobenzo(f)benzoxazolin-2-on, 10-Aminonaphthalino(g)benzoxazolin-2-on, Bis(4-aminobenzoxazolin-2-on-6-yl)-methan, Bis(5-aminobenzoxazolin-2-on-6-yl)-methan, Bis(7-aminobenzoxazolin-2-on-6-yl)-methan, Bis(4-aminobenzoxazolin-2-on-5-yl)-methan, Bis(6-aminobenzoxazolin-2-on-5-yl)-methan, Bis(7-aminobenzoxazolin-2-on-5-yl)-methan oder Bis(4-aminobenzoxazolin-2-on-6-yl)-propan.

Die Phosgenierung derartiger Amine kann nach an sich bekannten Verfahren zur Phosgenierung aromatischer Amine erfolgen und ist z. B. in Houben-Weyl, « Methoden der organischen Chemie », Band VIII/3, Seiten 119 ff., Thieme-Verlag, Stuttgart, beschrieben. Als Lösungsmittel für die Phosgenierung kommen Dioxan und andere cyclische Ether in Frage, daneben noch Chlorbenzol, Nitrobenzol oder Toluol. Die maximale Temperatur bei der Phosgenierungsreaktion sollte 130 °C nicht übersteigen. Vorzugsweise wird die Chlorwasserstoffabspaltung bei der Phosgenierungsreaktion im Temperaturbereich zwischen 80 und 120 °C durchgeführt.

Die Zugänglichkeit der erfindungsgemäßen Verbindungen durch Phosgenierung der ihnen zugrundeliegenden Amine ist überraschend, da gemäß DE-OS 1 595 690 Isocyanate mit Oxazolin-2-onen an der NH-Gruppe des Oxazolin-2-on-Rings unter Allophanatbildung reagieren, ein Befund, der durch die Untersuchungen von B. Krieg und H. Lautenschläger (Liebigs Annalen der Chemie, (1976), Seiten 208-211) an Benzoxazolin-2-onen bestätigt wurde. Es war somit damit zu rechnen, daß bei der Einwirkung von Phosgen auf die Amine polymere Allophanate, nicht aber die monomeren Isocyanato-oxazolin-2-one entstehen würden.

Bei der Phosgenierung der genannten Amine entstehen die erfindungsgemäßen Isocyanato-oxazolinone der obengenannten allgemeinen Formel. Bei den erfindungsgemäßen Verbindungen handelt es sich beispielsweise um 4-Isocyanatobenzoxazolin-2-on, 5-Isocyanatobenzoxazolin-2-on, 6-Isocyanatobenzoxazolin-2-on, 7-Isocyanatobenzoxazolin-2-on, 4-Isocyanato-6-methyl-benzoxazolin-2-on, 5-Isocyanato-6-methyl-benzoxazolin-2-on, 5-Isocyanato-6-chlorbenzoxazolin-2-on, 9-Isocyanatobenzo(g)benzoxazolin-2-on, 5-Isocyanatobenzo(g)benzoxazolin-2-on, 4-Isocyanatobenzo(e)benzoxazolin-2-on, 8-Isocyanatobenzo(f)benzoxazolin-2-on, 10-Isocyanatonaphthalino(g)benzoxazolin-2-on ;

Bis(4-isocyanatobenzoxazolin-2-on-6-yl)-methan, Bis-(5-isocyanatobenzoxazolin-2-on-6-yl)-methan, Bis(7-isocyanatobenzoxazolin-2-on-6-yl)-methan, Bis(4-isocyanatobenzoxazolin-2-on-5-yl)-methan, Bis(6-isocyanatobenzoxazolin-2-on-5-yl)-methan, Bis(7-isocyanatobenzoxazolin-2-on-5-yl)-methan, Bis(4-isocyanatobenzoxazolin-2-on-6-yl)-propan oder Bis(4-isocyanatobenzoxazolin-2-on-5-yl)-propan.

Beim erfindungsgemäßen Verfahren zur Herstellung von Oxazolin-2-on-Ringen aufweisenden Kunststoffvorläufern werden die oben beschriebenen erfindungsgemäßen Isocyanato-oxazolinone, mit Verbindungen mit gegenüber Isocyanatgruppen reaktionsfähigen Gruppen im Sinne einer Isocyanat-Additionsreaktion umgesetzt.

Geeignete Reaktionspartner für die genannten Isocyanatooxazolinone sind Hydroxylgruppen und/oder Aminogruppen aufweisende Verbindungen der als Reaktionspartner für organische Polyisocyanate aus der Polyurethanchemie bekannten Art. Diese gegenüber Isocyanatgruppen reaktionsfähige Gruppen aufweisenden Kunststoffvorläufer weisen im allgemeinen 2 bis 8, vorzugsweise 2 bis 4 und besonders bevorzugt 2 bis 3 gegenüber Isocyanaten reaktionsfähige Wasserstoffatome auf und haben im allgemeinen ein Molekulargewicht zwischen 18 und 10 000, vorzugsweise zwischen 62 und 8 000 besonders bevorzugt zwischen 400 und 6 000.

Geeignete Reaktionspartner für die genannten Isocyanatooxazolin-2-one sind beispielsweise die aus der Polyurethanchemie an sich bekannten Kettenverlängerungsmittel mit 2 primären und/oder sekundären Aminogruppen bzw. mit 2 alkoholischen Hydroxylgruppen des Molekulargewichts 32 bis 400 wie z. B. Hydrazin, N,N'-Dimethylhydrazin, Ethylendiamin, Tetramethylendiamin, 1-Amino-3,3,5-trimethyl-5-aminomethyl-cyclohexan (« Isophorondiamin »), 2,4- und 2,6-Hexahydrotoluylendiamin sowie deren Gemische, Perhydro-2,4'- und 4,4'-diaminodiphenylmethan, p-Xylylendiamin, Bis-(3-aminopropyl)-methylamin, Carbodihydrazid, Oxalsäuredihydrazid, die Dihydrazide von Bernsteinsäure, Glutarsäure, Adipinsäure, Sebazinsäure, Hydracrylsäure und Terephthalsäure ; Semicarbazido-alkylen-hydrazide wie z. B. β-Semicarbazidopropionsäurehydrazid, 2,4- oder 2,6-Toluylendiamin oder 4,4'-Diaminodiphenylmethan, Ethylenglykol, Propylenglykol-(1,2) und -(1,3), Butylenglykol-(1,4) und -(2,3), Pentandiol-(1,5), Hexandiol-(1,6), Octandiol-(1,8), Neopentylglykol, 1,4-Bis-hydroxymethyl-cyclohexan, 2-Methyl-1,3-propandiol, Diethylenglykol, Triethylenglykol, Tetraethylenglykol, höhere Polyethylenglykole mit einem Molekulargewicht bis 400, Dipropylenglykol, höhere Polypropylenglykole mit einem Molekulargewicht bis 400, Dibutylenglykol, höhere Polybutylenglykole mit einem Molekulargewicht bis 400, Bisphenol A, Di-(hydroxymethyl)-hydrochinon, Ethanolamin und 3-Aminopropanol.

Auch höherfunktionelle, niedermolekulare Alkohole wie z. B. Glycerin, Trimethylolpropan, Hexantriol-(1,2,6), Trimethylolethan oder Rizinusöl können beim erfindungsgemäßen Verfahren eingesetzt werden.

Zu den bevorzugten Reaktionspartnern für die genannten Isocyanato-oxazolin-2-one gehören die aus der Polyurethanchemie an sich bekannten Polyhydroxypolyester und/oder Polyhydroxypolyether der genannten Funktionalität und des genannten Molekulargewichtsbereichs.

Die in Frage kommenden Hydroxylgruppen aufweisenden Polyester sind z. B. Umsetzungsprodukte von mehrwertigen, vorzugsweise zweiwertigen und gegebenenfalls zusätzlich dreiwertigen Alkoholen mit mehrwertigen, vorzugsweise zweiwertigen, Carbonsäuren. Anstelle der freien Polycarbonsäuren können auch die entsprechenden Polycarbonsäureanhydride oder entsprechende Polycarbonsäureester von niedrigen Alkoholen oder deren Gemische zur Herstellung der Polyester verwendet werden. Die Polycarbonsäuren können aliphatischer, cycloaliphatischer, aromatischer und/oder heterocyclischer Natur sein und gegebenenfalls, z. B. durch Halogenatome, substituiert und/oder ungesättigt sein.

Als Beispiele für solche Carbonsäuren und deren Derivate seien genannt :

Bernsteinsäure, Adipinsäure, Phthalsäure, Isophthalsäure, Phthalsäureanhydrid, Tetrahydrophthalsäureanhydrid, Hexahydrophthalsäureanhydrid, Tetrachlorphthalsäureanhydrid, dimerisierte und trimerisierte ungesättigte Fettsäuren, gegebenenfalls in Mischung mit monomeren ungesättigten Fettsäuren, wie Ölsäure ; Terephthalsäuredimethylester und Terephthalsäure-bis-glykolester. Als mehrwertige Alkohole kommen beispielsweise die bereits oben beispielhaft genannten Verbindungen in Betracht.

Die als Reaktionspartner für die Isocyanato-oxazolin-2-one geeigneten Polyether werden in an sich bekannter Weise z. B. durch Polymerisation von Epoxiden wie Ethylenoxid, Propylenoxid, Butylenoxid, Tetrahydrofuran, Styroloxid oder Epichlorhydrin mit sich selbst, z. B. in Gegenwart von Lewis-Katalysatoren wie $BF_3$, oder durch Anlagerung dieser Epoxide, vorzugsweise von Ethylenoxid und Propylenoxid, gegebenenfalls im Gemisch oder nacheinander, an Startkomponenten mit reaktionsfähigen Wasserstoffatomen wie Wasser, Alkohole, Ammoniak oder Amine, z. B. Ethylenglykol, Propylenglykol-(1,3) oder -(1,2), Trimethylolpropan, Glycerin, Sorbit, 4,4'-Dihydroxy-diphenylpropan, Anilin, Ethanolamin oder Ethylendiamin hergestellt. Auch Sucrosepolyether, wie sie z. B. in den DE-Auslegeschriften 1 176 358 und 1 064 938 beschrieben werden, sowie auf Formit oder Formose gestartete Polyether (DE-Offenlegungsschriften 2 639 083 bzw. 2 737 951), kommen erfindungsgemäß in Frage. Vielfach sind solche Polyether bevorzugt, die überwiegend (bis zu 90 Gew.-%, bezogen auf alle vorhandenen OH-Gruppen im Polyether) primäre OH-Gruppen aufweisen. Auch OH-Gruppen aufweisende Polybutadiene sind erfindungsgemäß geeignet.

Auch höhermolekulare Polyamine der o. g. Funktionalität und des o. g. Molekulargewichtsbereichs können als Reaktionspartner für die Isocyanato-oxazolin-2-one eingesetzt werden. Es handelt sich hierbei beispielsweise um Anthranilsäureester, wie sie durch Umsetzung der oben beispielhaft genannten

höhermolekularen Polyhydroxylverbindungen mit Isatosäureanhydrid gemäß DE-OS 2 019 432 oder 2 619 840 bzw. gemäß US-PS 3 808 250, 3 975 428 oder 4 016 143 erhalten werden ; oder um Verbindungen, die durch Umsetzung von NCO-Präpolymeren mit Hydroxylgruppen aufweisenden Enaminen, Aldiminen oder Ketiminen und anschließender Hydrolyse gemäß DE-OS 2 546 536 bzw. US-PS 3 865 791 erhalten worden sind ; oder um Polyamine, die durch Hydrolyse der NCO-Gruppen von NCO-Präpolymeren einer entsprechenden Funktionalität und eines entsprechenden Molekulargewichts erhalten werden können. Weitere Herstellungsverfahren für geeignete höhermolekulare Verbindungen mit endständigen Aminogruppen oder Hydrazidgruppen werden in der DE-OS 1 694 152 (US-PS 3 625 871) beschrieben.

Es ist auch möglich, beim erfindungsgemäßen Verfahren Polyhydroxylverbindungen einzusetzen, in welchen hochmolekulare Polyaddukte bzw. Polykondensate oder Polymerisate in feindisperser oder gelöster Form enthalten sind. Derartige Polyhydroxylverbindungen werden z. B. erhalten, wenn man Polyadditionsreaktionen (z. B. Umsetzungen zwischen Polyisocyanaten und aminofunktionellen Verbindungen) bzw. Polykondensationsreaktionen (z. B. zwischen Formaldehyd und Phenolen und/oder Aminen) in situ in den o. g., Hydroxylgruppen aufweisenden Verbindungen ablaufen läßt. Derartige Verfahren sind beispielsweise in den DE-Auslegeschriften 1 168 075 und 1 260 142, sowie den DE-Offenlegungsschriften 2 324 134, 2 423 984, 2 512 385, 2 513 815, 2 550 796, 2 550 797, 2 550 833, 2 550 862, 2 633 293 und 2 639 254 beschrieben. Es ist aber auch möglich, gemäß US-Patentschrift 3 869 413 bzw. DE-Offenlegungsschrift 2 550 860 eine fertige wäßrige Polymerdispersion mit einer Polyhydroxylverbindung zu vermischen und anschließend aus dem Gemisch das Wasser zu entfernen.

Auch durch Vinylpolymerisate modifizierte Polyhydroxylverbindungen, wie sie z. B. durch Polymerisation von Styrol und Acrylnitril in Gegenwart von Polyethern (US-Patentschriften 3 383 351, 3 304 273, 3 523 093, 3 110 695 ; DE-Auslegeschrift 1 152 536) oder Polycarbonatpolyolen (DE-Patentschrift 1 769 795 ; US-Patentschrift 3 637 909) erhalten werden, sind für das erfindungsgemäße Verfahren geeignet. Bei Verwendung von Polyetherpolyolen, welche gemäß den DE-Offenlegungsschriften 2 442 101, 2 644 922 und 2 646 141 durch Pfropfpolymerisation mit Vinylphosphonsäureestern sowie gegebenenfalls (Meth)acrylnitril, (Meth)acrylamid oder OH-funktionellen (Meth)acrylsäureestern modifiziert wurden, erhält man Kunststoffe von besonderer Flammwidrigkeit. Polyhydroxylverbindungen, in welche durch radikalische Pfropfpolymerisation mittels ungesättigter Carbonsäuren sowie gegebenenfalls weiterer olefinisch ungesättigter Monomerer Carboxylgruppen eingeführt wurden (DE-Offenlegungsschriften 2 714 291, 2 739 620 und 2 654 746), können mit besonderem Vorteil in Kombination mit mineralischen Füllstoffen eingesetzt werden.

Als Reaktionspartner für die genannten Isocyanatooxazolin-2-one können anstelle oder zusätzlich zu den genannten Verbindungen mit gegenüber Isocyanatgruppen reaktionsfähigen Wasserstoffatomen auch die aus der Polyurethanchemie bekannten, im allgemeinen jedoch weniger bevorzugten Polythioether, Polyacetale, Polycarbonate oder Polyesteramide mit endständigen Hydroxylgruppen oder auch bereits Urethan- oder Harnstoffgruppen enthaltende Polyhydroxylverbindungen eingesetzt werden.

Selbstverständlich können beim erfindungsgemäßen Verfahren beliebige Gemische der beispielhaft genannten Verbindungen mit gegenüber Isocyanatgruppen reaktionsfähigen Gruppen, insbesondere Gemische aus höhermolekularen Polyhydroxylverbindungen und niedermolekularen Kettenverlängerungsmitteln, insbesondere niedermolekularen Alkoholen, verwendet werden.

Bei der Durchführung des erfindungsgemäßen Verfahrens können neben den Isocyanato-oxazolinonen und den Verbindungen mit gegenüber Isocyanatgruppen reaktionsfähigen Gruppen gegebenenfalls noch weitere, Oxazolinongruppen-freie Polyisocyanate eingesetzt werden. Hierbei handelt es sich um die aus der Polyurethanchemie bekannten Polyisocyanate, beispielsweise der Formel

$$Q(NCO)_n$$

in der

n 2 bis 4, vorzugsweise 2 und

Q einen aliphatischen Kohlenwasserstoffrest mit 2 bis 18, vorzugsweise 6 bis 10, C-Atomen, einen cycloaliphatischen Kohlenwasserstoffrest mit 4 bis 15, vorzugsweise 5 bis 10 C-Atomen, einen aromatischen Kohlenwasserstoffrest mit 6 bis 15, vorzugsweise 6 bis 13, C-Atomen, oder einen araliphatischen Kohlenwasserstoffrest mit 8 bis 15, vorzugsweise 8 bis 13 C-Atomen, bedeuten, z. B. 1,6-Hexamethylendiisocyanat, 1,12-Dodecandiisocyanat, 1-Isocyanato-3,3,5-trimethyl-5-isocyanatomethyl-cyclohexan, Perhydro-2,4′-und/oder -4,4-diphenylmethan-diisocyanat, 2,4- und 2,6-Toluylendiisocyanat sowie beliebige Gemische dieser Isomeren, Diphenylmethan-2,4′- und/oder -4,4′-diisocyanat, Naphthylen-1,5-diisocyanat. Ferner kommen beispielsweise erfindungsgemäß in Frage : Polyphenyl-polymethylenpolyisocyanate, wie sie durch Anilin-Formaldehyd-kondensation und anschließende Phosgenierung erhalten und z. B. in den GB-Patentschriften 874 430 und 848 671 beschrieben werden, oder insbesondere freie Isocyanatgruppen aufweisende Prepolymere, wie sie in an sich bekannter Weise durch Umsetzung von einfachen organischen Polyisocyanaten der beispielhaft genannten Art mit unterschüssigen Mengen an organischen Polyhydroxylverbindungen der oben beispielhaft genannten Art erhalten werden. Diese NCO-Präpolymeren weisen im allgemeinen einen NCO-Gehalt von 0,5 bis 26, vorzugsweise 1 bis 15 Gew.-% auf.

Bei der Durchführung des erfindungsgemäßen Verfahrens kommen die Reaktionspartner in den bereits o. g. Mengenverhältnissen zum Einsatz. Je nach Art und Mengenverhältnisse der beispielhaft

genannten Reaktionspartner entstehen beim erfindungsgemäßen Verfahren in der Hitze selbstvernetzende oder in Gegenwart von Kettenverlängerungs- bzw. Vernetzungsmitteln zu hochmolekularen Kunststoffen ausreagierbare Kunststoffvorläufer. Selbstvernetzende Kunststoffvorläufer liegen dann vor, wenn bei der Durchführung des erfindungsgemäßen Verfahrens entweder ein Isocyanat- oder ein Aminüberschuß eingesetzt wird, da dann Isocyanat- bzw. Aminogruppen und mit diesen Gruppen in der Hitze reaktionsfähige Oxazolin-2-on-Ringe vorliegen. Zur Herstellung von Isocyanatgruppen aufweisenden, selbstvernetzenden, erfindungsgemäßen Verfahrensprodukten setzt man die Isocyanato-oxazolin-2-one vorzugsweise mit überschüssigen Mengen an Polyhydroxylverbindungen der oben beispielhaft genannten Art um, so daß auf jede Isocyanatgruppe des Isocyanato-oxazolin-2-ons 1,5 bis 3, vorzugsweise 2 Hydroxylgruppen der Polyhydroxylverbindung entfallen. Die in diesem ersten Reaktionsschritt erhaltenen Hydroxylgruppen und Oxazolin-2-on-gruppen aufweisenden Verbindungen können dann in einem zweiten Reaktionsschritt mit Oxazolin-2-on-freien Polyisocyanaten der beispielhaft genannten Art zu Isocyanatgruppen, Urethangruppen und Oxazolin-2-on-gruppen aufweisenden Kunststoffvorläufern umgesetzt werden. Hierzu werden die überschüssige Hydroxylgruppen aufweisenden Umsetzungsprodukten des 1. Reaktionsschritts mit den Oxazolin-2-on-freien Polyisocyanaten vorzugsweise in solchen Mengenverhältnissen der Reaktionspartner umgesetzt, daß auf jede Hydroxylgruppe der genannten Umsetzungsprodukte bis zu 3, vorzugsweise 1,5 bis 2,5 und insbesondere 2, Isocyanatgruppen des Oxazolinon-freien Polyisocyanats entfallen.

Insbesondere bei Verwendung von organischen Polyaminen der oben beispielhaft genannten Art als Reaktionspartner für die Isocyanato-oxazolin-2-one werden diese oftmals im Überschuß eingesetzt, so daß Aminogruppen, Harnstoffgruppen und Oxazolin-2-on-gruppen aufweisende Kunststoffvorläufer entstehen, die dann, wie bereits dargelegt, wegen des gleichzeitigen Vorliegens von Aminogruppen und Oxazolin-2-ongruppen selbstvernetzend sind. Bei dieser Ausführungsform des erfindungsgemäßen Verfahrens werden die Reaktionspartner in solchen Mengenverhältnissen eingesetzt, daß auf jede Isocyanatgruppe des Isocyanato-oxazolin-2-ons bis zu 3, vorzugsweise 1,5 bis 2,5 und insbesondere 2 Aminogruppen des Polyamins entfallen.

Gemäß einer dritten Ausführungsform des erfindungsgemäßen Verfahrens kommen die Isocyanato-oxazolin-2-one und die Verbindungen mit gegenüber Isocyanatgruppen reaktionsfähigen Gruppen in äquivalenten bzw. nahezu äquivalenten Mengen zum Einsatz, d. h. in solchen Mengen, daß auf jede Isocyanatgruppe des Isocyanato-oxazolin-2-ons 0,95 bis 1,1 gegenüber Isocyanatgruppen reaktionsfähige Gruppen entfallen. Im übrigen werden Art und Mengenverhältnisse der beim erfindungsgemäßen Verfahren zum Einsatz gelangenden Ausgangsmaterialien stets so bemessen, daß in den erfindungsgemäßen Verfahrensprodukten im statistischen Mittel pro Molekül 2 bis 6 Oxazolin-2-on-Ringe vorliegen.

Falls selbstvernetzende Verfahrensprodukte der genannten Art angestrebt werden, werden Art und Mengenverhältnisse der Reaktionspartner vorzugsweise so gewählt, daß in den Verfahrensprodukten auf jede Oxazolin-2-on-Gruppe 0,6 bis 1,4, vorzugsweise 1 Amino- bzw. Isocyanatgruppe entfallen.

Das erfindungsgemäße Verfahren wird im Temperaturbereich zwischen 20 und 100 °C, bevorzugt zwischen 60 und 90 °C in Substanz oder in Anwesenheit inerter Lösungsmittel durchgeführt. Geeignete Lösungsmittel sind beispielsweise Dioxan, Benzol, Toluol, Xylol, Chlorbenzol, Nitrobenzol, Ethylenglykolmonomethylether-acetat oder Tetrahydrofuran bzw. Gemische derartiger Lösungsmittel. Die Reaktionstemperatur muß selbstverständlich so gewählt werden, daß in den erfindungsgemäßen Verfahrensprodukten die Oxazolin-2-on-Ringe unverändert vorliegen.

Wie bereits ausgeführt, entstehen beim erfindungsgemäßen Verfahren Umsetzungsprodukte, welche aromatische Ringsysteme mit ankondensierten Oxazolin-2-on-Ringen enthalten, wobei diese Ringsysteme mit den organischen Resten der o. g. Reaktionspartner über Harnstoffgruppierungen (im Falle der Verwendung von Polyaminen), oder Urethangruppierungen (im Falle der Verwendung von Polyhydroxylverbindungen) verknüpft sind. Die dabei ablaufenden Additionsreaktionen können durch folgende Gleichungen verdeutlicht werden.

a) Reaktionen mit org. Polyaminen gemäß

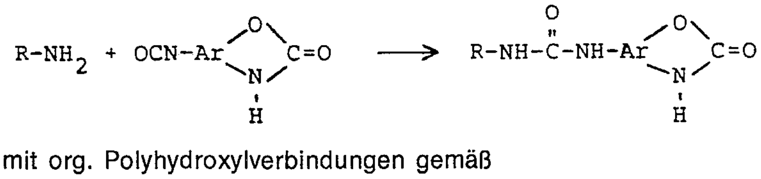

b) Reaktionen mit org. Polyhydroxylverbindungen gemäß

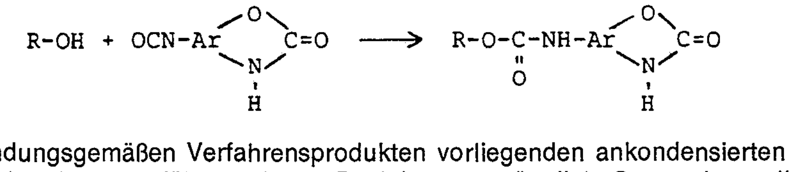

Die in den erfindungsgemäßen Verfahrensprodukten vorliegenden ankondensierten Oxazolin-2-on-Ringe sind nun, wie bereits ausgeführt, weiteren Reaktionen zugänglich. So reagieren die am Stickstoff des Oxazolin-2-on-Ring gebundenen Wasserstoffatome mit org. Isocyanaten gemäß

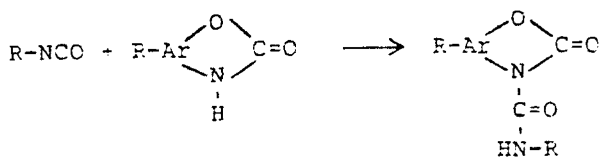

oder mit Epoxiden gemäß

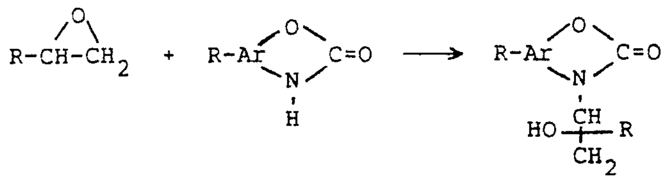

Mit primären oder sekundären Aminen reagieren die Oxazolin-2-on-Ringe gemäß folgendem Formelschema :

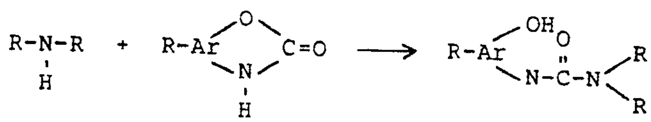

In diesen, lediglich zur Verdeutlichung der einzelnen Reaktionsmechanismen dienenden Gleichungen stehen die Reste R und Ar für die, bezüglich der Umsetzungen indifferenten, Reste der jeweiligen Reaktionspartner.

Als bei der erfindungsgemäßen Verwendung gegebenenfalls mitzuverwendende Vernetzungs- bzw. Kettenverlängerungsmittel eignen sich

1. die oben beispielhaft genannten organischen Polyisocyanate, insbesondere die oben beispielhaft genannten NCO-Präpolymeren,

2. Polyepoxide wie z. B. Glycidylether, die durch Epoxidierung der entsprechenden Allylether oder nach bekannten Verfahren durch Umsetzung eines molaren Überschusses von Epichlorhydrin mit einer aromatischen Polyhydroxyverbindung, wie z. B. Isopropylidenbisphenol, einem Novolak, Resorcin oder ähnlichen hergestellt werden. Besonders bevorzugt sind die Epoxidderivate von Methylen- oder Isopropylidenbisphenolen. In den nachfolgenden Beispielen wurde als Epoxidharz der Diglycidylether von 4,4′-Isopropylidenbisphenol eingesetzt.

Eine verbreitet angewandte Gruppe von gemäß der Erfindung geeigneten Polyepoxiden sind die harzartigen Epoxidpolyether, die durch Umsetzung eines Epihalohydrins wie Epichlorhydrin u. ä. mit einem Polyhydroxyphenol oder einem Polyhydroxyalkohol erhalten werden. Die Epoxidharze haben durchschnittlich mindestens 2 reaktive 1,2-Epoxidgruppen pro Molekül. Beispiele für geeignete Dihydroxyphenole sind : 4,4′-Isopropylidenbisphenol, 2,4′-Dihydroxydiphenylethylmethan, 3,3′-Dihydroxydiphenyldiethylmethan, 3,4′-Dihydroxydiphenylmethylpropylmethan, 2,3′-Dihydroxydiphenylethylphenylmethan, 4,4′-Dihydroxydiphenylpropylphenylmethan, 4,4′-Dihydroxydiphenylbutylphenylmethan, 2,2′-Dihydroxydiphenylditolylmethan, 4,4′-Dihydroxydiphenyltolylmethan und ähnliche.

Andere Polyhydroxyphenole, die ebenfalls mit einem Epihalohydrin zur Herstellung dieser Epoxidpolyether umgesetzt werden können, sind Verbindungen, wie Resorcin, Hydrochinon, substituierte Hydrochinone, wie z. B. Methylhydrochinon und ähnliche.

Unter den Polyhydroxyalkoholen, die mit einem Epihalohydrin zur Herstellung dieser harzartigen Epoxidpolyether umgesetzt werden können, sind Verbindungen wie Ethylenglykol, Propylenglykole, Butylenglykole, Pentandiole, Bis-(4-hydroxycyclohexyl)-dimethylmethan, 1,4-Dimethylolbenzol, Glycerin, 1,2,6-Hexantriol, Trimethylolpropan, Mannit, Sorbit, Erythrit, Pentaerythrit, Dimere, Trimere und höhere Polymere derselben, wie z. B. Polyethylenglykole, Polypropylenglykole, Triglycerin oder Dipentaerythrit, Polyallylalkohol, Polyhydroxythioether, wie z. B. 2,2′, 3,3′-Tetrahydroxydipropylsulfid, Mercaptoalkohole wie Monothioglycerin oder Dithioglycerin, partiell veresterte Polyhydroxyalkohole wie Monostearin wie Monostearin oder Pentaerythritmonoacetat und halogenierte Polyhydroxyalkohole wie die Monochlorhydrine von Glycerin, Sorbit oder Pentaerythrit.

Eine andere Gruppe von polymeren Polyepoxiden, die gemäß der Erfindung angewendet und durch Amin gehärtet werden können, sind die Epoxid-Novolak-Harze, die durch Umsetzung eines Epihalohydrins, wie Epichlorhydrin, mit dem harzartigen Kondensat eines Aldehyds, z. B. Formaldehyd mit entweder einem Monohydroxyphenol, wie z. B. Phenol selbst oder einem Polyhydroxyphenol vorzugsweise in Gegenwart eines basischen Katalysators, wie z. B. Natrium- oder Kaliumhydroxid, erhalten werden. Weitere Einzelheiten über die Eigenschaften und Herstellung dieser Epoxid-Novolak-Harze können aus H. Lee und K. Neville, Handbook of Epoxy Resins, McGraw Hill Book Co., New York, 1967, entnommen werden.

3. Organische Polyamine, d. h. insbesondere aliphatische diprimäre Diamine mit 2 bis 12 Kohlenstoffatomen, diprimäre cycloaliphatische Diamine mit 6 bis 15 Kohlenstoffatomen, diprimäre aromatische Diamine mit 6 bis 15 Kohlenstoffatomen wie z. B. Ethylendiamin, Tetramethylendiamin, Hexamethylendiamin, Undecamethylendiamin, Dodecamethylendiamin, 1-Amino-3,3,5-trimethyl-5-amino-methylcyclohexan, 2,4- und 2,6-Hexahydrotoluylendiamin sowie deren Gemische, Perhydro-2,4'- und 4,4'-diaminodiphenylmethan, p-Xylylendiamin, Bis-(3-aminopropyl)-methylamin, 4,4'-Diaminodicyclohexylmethan, 3,3'-Dimethyl-4,4'-diamino-dicyclohexylmethan, 3,5,3',5'-Tetraethyl-4,4'-diaminodicyclohexylmethan, 2,2'-Bis-(4-aminocyclohexyl)-propan, 2,4- bzw. 2,6-Diaminotoluol oder 4,4'-Diaminodiphenylmethan. Organische Polyamine, die beispielsweise durch Hydrolyse der NCO-Gruppen der oben beispielhaft genannten NCO-Präpolymeren erhalten werden können, sind ebenfalls als erfindungsgemäße Kettenverlängerungs- bzw. Vernetzungsmittel geeignet. Außerdem können cycloaliphatische Triamine beispielsweise solche gemäß DE-OS 2 614 244 oder sekundäre Amine wie z. B. Piperazin oder N,N',N"-Trimethyldiethylentriamin als erfindungsgemäß verwendbare Vernetzer bzw. Kettenverlängerungsmittel eingesetzt werden.

Bei der erfindungsgemäßen Verwendung der erfindungsgemäßen Verfahrensprodukte werden die Vernetzungs-bzw. Kettenverlängerungsmittel im allgemeinen in solchen Mengen eingesetzt, daß auf jede gegenüber dem Vernetzungsmittel reaktionsfähige Gruppe der Verfahrensprodukte (der Oxazolin-2-on-Ring stellt hierbei eine monofunktionelle Gruppe dar) 0,95 bis 1,5, vorzugsweise 0,95 bis 1,05 reaktive Gruppen des Vernetzungs- bzw. Kettenverlängerungsmittels entfallen. Die Menge des Vernetzungsmittels kann, diesen Ausführungen entsprechend, je nach Gehalt der erfindungsgemäßen Verfahrensprodukte an Isocyanat- bzw. Aminogruppen verringert werden, so daß insbesondere bei Vorliegen äquivalenter Mengen an Amino- oder Isocyanatgruppen und Oxazolin-2-on-Ringen im Verfahrensprodukt auf den Einsatz der Vernetzungs- bzw. Kettenverlängerungsmittel völlig verzichtet werden kann.

Die Temperatur, bei welcher die Vernetzungs- bzw. Kettenverlängerungsreaktion abläuft, hängt in erster Linie von der Natur des Vernetzungs- bzw. Kettenverlängerungsmittels ab. Sie liegt im Falle von organischen Polyisocyanaten und organischen Polyaminen im allgemeinen im Temperaturbereich zwischen 100 und 180 °C, vorzugsweise 120 bis 160 °C. Bei Verwendung von mindestens difunktionellen Polyepoxiden liegt die Vernetzungs- bzw. Kettenverlängerungstemperatur im allgemeinen innerhalb des Temperaturbereichs von 140 bis 200, vorzugsweise 150 bis 180 °C.

Sowohl bei der Durchführung des erfindungsgemäßen Verfahrens als auch bei der erfindungsgemäßen Verwendung der erfindungsgemäßen Verfahrensprodukte können, die jeweilige Reaktion beschleunigende Katalysatoren mitverwendet werden. So empfiehlt sich beispielsweise bei der Herstellung von Harnstoffgruppen aufweisenden erfindungsgemäßen Verfahrensprodukten unter Verwendung von organischen Polyisocyanaten als Ausgangsmaterialien oft der Einsatz der in der Polyurethanchemie üblichen tertiären Amine als Katalysatoren. Beispiele derartiger Katalysatoren sind Triethylendiamin oder N,N-Dimethylbenzylamin. Geeignete Katalysatoren für die Vernetzungs- bzw. Kettenverlängerungsreaktion unter Verwendung von organischen Polyaminen sind beispielsweise Titan-tetraalkylate wie z. B. Titan-tetrabutylat oder Titan-tetraethylat.

Auch die Herstellung von hochmolekularen Kunststoffen unter erfindungsgemäßer Verwendung der erfindungsgemäßen Verfahrensprodukte kann in Gegenwart und in Abwesenheit geeigneter inerter Lösungsmittel der oben bereits beispielhaft genannten Art erfolgen.

Auch die Mitverwendung von Treibmitteln wie z. B. von in der Hitze gasabspaltenden Verbindungen wie Azocarbonamid ist möglich. In diesem Falle können bei der erfindungsgemäßen Verwendung geschäumte Kunststoffe erhalten werden. Falls als Vernetzungs- bzw. Kettenverlängerungsmittel organische Polyisocyanate eingesetzt werden, kann selbstverständlich auch Wasser als Treibmittel verwendet werden.

Durch geeignete Wahl der Ausgangsmaterialien bei der Durchführung des erfindungsgemäßen Verfahrens und auch durch geeignete Wahl der bei der erfindungsgemäßen Verwendung eingesetzten Vernetzungs- bzw. Kettenverlängerungsmittel können die mechanischen Eigenschaften der letztlich erhaltenen Kunststoffe wie beispielsweise deren Härte und Elastizität dem jeweiligen Verwendungszweck in optimaler Weise angepaßt werden. So kann beispielsweise die Elastizität der erfindungsgemäß erhältlichen Kunststoffe durch den Einbau langkettiger Polyester- bzw. Polyethersegmente erhöht werden. Die erfindungsgemäß erhältlichen Kunststoffe können zur Herstellung von massiven Formteilen, als Klebstoffe, als Schaumkunststoffe oder als Beschichtungen beliebiger Substrate Verwendung finden.

In den nachfolgenden Beispielen beziehen sich alle Prozentangaben auf Gewichtsprozente.

Allgemeines Verfahren zur Herstellung der erfindungsgemäßen Isocyanato-oxazolin-2-one

Zur Herstellung der erfindungsgemäßen Isocyanato-oxazolin-2-one geht man von den entsprechenden Aminen aus, die nach den oben angegebenen Methoden gut zugänglich sind.

Bei der Phosgenierung wird im allgemeinen so vorgegangen, daß 1 bis 3 Mol Phosgen in 1 l wasserfreiem Dioxan bei Raumtemperatur vorgelegt wird. Das in das Isocyanat zu überführende Amin wird dann als Lösung oder Suspension in Dioxan zugegeben. Die Phosgenvorlage wird dabei mit Eis gekühlt, die Innentemperatur sollte nicht über 20 °C steigen. Auf 3 Mol vorgelegtes Phosgen wird ca. 1 Mol Amin eingesetzt. Nach vollständiger Aminzugabe wird noch 1 Stunde bei Raumtemperatur gerührt und dann langsam auf 100 °C erhitzt, wobei vor Erreichen dieser Temperatur starke HCl-Entwicklung zu

beobachten ist (im allgemeinen bei ca. 60 bis 80 °C). Nach 6 bis 10 Stunden ist das Carbomoylchlorid vollständig in Lösung gegangen, worauf überschüssiges Phosgen mit Stickstoff ausgeblasen wird. Die im allgemeinen nur geringfügige unlösliche Reste aufweisende Lösung wird filtriert und das Lösungsmittel abdestilliert. Das resultierende Produkt ist für gewöhnlich für weitere Umsetzungen rein genug, insbesondere deuten die NCO-Werte auf vollständige Umsetzung. Für analytische Zwecke kann aus Solventien wie Dioxan, Toluol, Aceton umkristallisiert werden, die Ausbeuten sind gut bis quantitativ.

Beispiel 1

6-Isocyanatobenzoxazolin-2-on

190 g (1,9 Mol) Phosgen werden in 1 l trockenem Dioxan gelöst und vorgelegt. 93 g (0,62 Mol) 6-Aminobenzoxazolin-2-on werden als Suspension in 800 ml Dioxan zugegeben (45 Minuten, 20 °C) und danach noch 1 Stunde bei 25 °C gerührt und anschließend noch 3 Stunden unter Überleitung eines leichten Phosgen-Stromes bei 90 °C gerührt. Ab 80 °C setzte starke Gasentwicklung ein. Überschüssiges Phosgen wurde mit trockenem Stickstoff ausgetrieben. Von geringen Mengen unlöslichen Rückstands wurde abfiltriert und das Solvens abdestilliert. Es resultierten 111 g (100 %) an 6-Isocyanatobenzoxazolin-2-on mit Schmp. 130 °C (unter Zersetzung) und einem NCO-Gehalt von 25,9 % (Theorie : 23,8 %).

Bei der Schmelzpunktbestimmung zeigte sich, daß die Schmelze sich wieder verfestigte, ohne bis 300 °C erneut zu schmelzen.

Beispiel 2

Entsprechend Beispiel 1 wurde 5-Isocyanato-benzoxazolin-2-on aus dem entsprechenden Amin in 98,5 %iger Ausbeute erhalten. Das Produkt weist einen über 350 °C liegenden Schmelzpunkt und einen NCO-Gehalt von 23,3 % (Theorie : 23,8 %) auf.

Beispiel 3

Entsprechend Beispiel 1 wurde 6-Methyl-4-isocyanatobenzoxazolin-2-on aus dem entsprechenden Amin in 74 %iger Ausbeute erhalten. Das Produkt weist einen Schmelzpunkt von 182-183 °C und einen NCO-Gehalt von 19 % (Theorie : 22,1 %) auf.

Beispiel 4

Entsprechend Beispiel 1 wurde 5-Isocyanato-6-chlorbenzoxazolin-2-on aus dem entsprechenden Amin in 98,4 %iger Ausbeute erhalten. Das Produkt weist einen Schmelzpunkt von 146 °C und einen NCO-Gehalt von 15,3 % (Theorie : 20 %) auf.

Beispiel 5

Bis-(4-isocyanatobenzoxazolin-2-on-5-yl)-methan

188 g (1,9 Mol) Phosgen werden in 1,5 l Dioxan gelöst und vorgelegt. Bei einer Temperatur von 10 °C wird eine Suspension von 90 g (0,29 Mol) Bis-(4-aminobenzoxazolin-2-on-5-yl)-methan in 600 ml Dioxan hinzugegeben und 1 Stunde anschließend bei 20 °C weitergerührt. Danach wird unter Rühren langsam auf 85 °C erhitzt, wobei ab 40 °C ein schwacher Phosgenstrom übergeleitet wird und HCl-Abspaltung ab 60 °C beginnt. Nach 4-stündigem Erhitzen auf 85 °C wird überschüssiges Phosgen mit Stickstoff ausgetrieben, von unlöslichen Begleitmaterialien abfiltriert und das Lösungsmittel abdestilliert. Der verbliebene Rückstand wird bei 50 °C im Hochvakuum getrocknet.

Es resultieren 80 g (76 % der Theorie) eines Produkts mit Schmp. 163 bis 166 °C und einem NCO-Gehalt von 21 % (Theorie : 23 %).

Beispiel 6

Entsprechend dem vorgehenden Beispiel wird Bis-(5-isocyanatobenzoxazolin-2-on-6-yl)-methan durch Phosgenierung des entsprechenden Amins erhalten. Das Produkt weist einen Schmelzpunkt von 148 bis 150 °C und einen NCO-Gehalt von 21,7 % (Theorie : 23 %) auf.

Beispiel 7

30 g eines durch Hydrolyse eines aus Hexamethylendiisocyanat und Dipropylenglykol hergestellten NCO-Präpolymeren (NCO-Gehalt : 14 %) erhaltenen Diamins (NH-Zahl : 203) werden mit 8,2 g 5-Isocyanatobenzoxazolin-2-on intensiv vermischt ($NCO/NH_2$-Äquivalentverhältnis = 1 : 2) und während 1 Stunde auf 80 °C erhitzt. Das hierbei entstehende Amino- und Oxazolin-2-on-Gruppen aufweisende Umsetzungsprodukt kann durch 2-stündiges Erhitzen auf 130 °C zu einem festen spröden Kunststoff umgesetzt werden.

Beispiel 8

30 g eines durch Hydrolyse eines aus Hexamethylendiisocyanat und Trimethylhexandiol-1,6 hergestellten NCO-Präpolymeren (NCO-Gehalt : 15,4 %) erhaltenen Diamins (NH-Zahl : 218) werden mit 8,75 g 5-Isocyanatobenzoxazolin-2-on intensiv vermischt und während 1 Stunde auf 80 °C erhitzt ($NCO/NH_2$-Äquivalentverhältnis = 1 : 2). Das dabei entstehende Aminogruppen und Benzoxazolin-2-on-gruppen aufweisende Umsetzungsprodukt kann durch 2-stündiges Erhitzen auf 130 °C zu einem festen, spröden Kunstoff vernetzt werden.

Beispiel 9

30 g eines durch Hydrolyse eines aus Hexamethylendiisocyanat und Polypropylenglykol der OH-Zahl 112 hergestellten NCO-Präpolymeren (NCO-Gehalt : 6,3 %) erhaltenen Diamins (NH-Zahl : 79,4) werden mit 3,69 g 5-Isocyanatobenzoxazolin-2-on intensiv vermischt ($NCO/NH_2$-Äquivalentverhältnis = 1 : 2) und bei 90 °C/1 h zu einer hochviskosen, in Butylacetat gut löslichen, Substanz umgesetzt, die sich durch 2-stündiges Erhitzen auf 130 °C in einen weichen Kunststoff umwandeln läßt.

Beispiel 10

20 g einer 1 : 1-Mischung aus einem Diamin der NH-Zahl 203, hergestellt durch Hydrolyse eines aus Hexamethylendiisocyanat und Dipropylenglykol gewonnenen NCO-Präpolymeren (NCO-Gehalt : 14 %), und einem Diamin der NH-Zahl 79,4, hergestellt durch Hydrolyse eines aus Polypropylenglykol der OH-Zahl 112 und Hexamethylendiisocyanat gewonnenen NCO-Präpolymeren (NCO-Gehalt : 6,3 %) werden mit 3,9 g 5-Isocyanatobenzoxazolin-2-on intensiv vermischt und bei 90 °C/1 h zu einem harzartigen Produkt umgesetzt, das durch 3-stündiges Erhitzen auf 130 °C zu einem festen, wenig elastischen Kunststoff ausgehärtet werden kann.

Beispiel 11

38 g (0,2 Mol) 5-Methyl-7-isocyanatobenzoxazolin-2-on werden 4 Stunden bei 80 °C mit 100 g (0,1 Mol) Polypropylenglykol der OH-Zahl 112 zur Umsetzung gebracht. Es entsteht eine NCO-freie, hochviskose Flüssigkeit, die bei 50 °C gut verarbeitbar ist.

50 g des genannten Umsetzungsproduktes werden mit 2,1 g Hexamethylendiamin (Äquivalentverhältnis zwischen Aminogruppen und Benzoxazolin-2-on-Gruppen = 1 : 1) intensiv vermischt und bei 140 °C/2 h zu einem wachsartigen Kunststoff umgesetzt.

Völlig entsprechend kann das Umsetzungsprodukt auch mit einer äquivalenten Menge an 2,4-Diaminotoluol bei 140 °C/2 h zu einem wachsartigen Kunststoff umgesetzt werden.

Beispiel 12

20 g (0,114 Mol) 6-Isocyanatobenzoxazolin-2-on werden unter leichtem Anwärmen in 250 ml wasserfreiem Dioxan gelöst. 57 g Polypropylenglykol der OH-Zahl 112 werden zugesetzt. Das Reaktionsgemisch wird während 2 Stunden bei 80 °C gerührt. Nach Abdestillieren des Dioxans resultiert eine hochviskose, NCO- und $H_2N$-freie Oxazolin-2-on-Gruppen aufweisende Substanz.

110 g (0,007 4 Mol) dieser Substanz werden mit 4,7 g eines durch Hydrolyse eines NCO-Präpolymeren aus Hexamethylendiisocyanat und Dipropylenglykol hergestellten NCO-Präpolymeren (NCO-Gehalt : 14 %) hergestellten Diamins (NH-Zahl : 203) intensiv vermischt und bei 130 °C/3 h zu einem weichen, leicht klebrigen Kunststoff umgesetzt.

Beispiel 13

25 g (0,014 2 Mol) 5-Isocyanatobenzoxazolin-2-on werden mit 142 g Polypropylenglykol der OH-Zahl 56 während 3 Stunden bei 90 °C zu einer hochviskosen, NCO- und $H_2N$-Gruppen-freien, Oxazolin-2-on-Gruppen aufweisenden Substanz umgesetzt.

26,6 g dieser Verbindung werden mit 100 g eines durch Hydrolyse eines NCO-Präpolymeren aus Hexamethylendiisocyanat und Dipropylenglykol hergestellten NCO-Präpolymeren (NCO-Gehalt : 14 %) erhaltenen Diamins (NH-Zahl : 203) intensiv vermischt und bei 130 °C/2 h zu einem weichen Kunststoff umgesetzt.

Beispiel 14

20 g (0,114 Mol) 6-Isocyanatobenzoxazolin-2-on werden unter leichtem Erwärmen in 250 ml wasserfreiem Dioxan gelöst. 57 g Polypropylenglykol der OH-Zahl 112 werden zugesetzt. Anschließend wird während 2 Stunden bei 80 °C gerührt. Nach destillativer Entfernung des Dioxans verbleibt eine NCO-freie hochviskose Substanz.

10 g (0,007 4 Mol) dieser Substanz werden mit 2,7 g eines NCO-Präpolymeren aus 4,4'-Diisocyanatodiphenylmethan und Dipropylenglykol (NCO-Gehalt: 22 %) intensiv vermischt und durch 3-stündiges Erhitzen auf 130 °C zu einem festen Kunststoff ausgehärtet.

Beispiel 15

25 g (0,142 Mol) 5-Isocyanatobenzoxazolin-2-on werden mit 142 g Polypropylenglykol der OH-Zahl 56 durch 3-stündiges Erhitzen auf 90 °C zu einer hochviskosen NCO- und $H_2N$-freien, Oxazolin-2-ongruppen aufweisenden Substanz umgesetzt.

20 g (0,008 5 Mol) dieser Verbindung werden mit 3,15 g eines NCO-Präpolymeren aus 4,4'-Diisocyanatodiphenylmethan und Dipropylenglykol (NCO-Gehalt: 22 %) intensiv vermischt und durch 4-stündiges Erhitzen auf 130 °C zu einem sehr weichen Kunststoff umgesetzt.

In einem weiteren Versuch wurden 23,5 g (0,01 Mol) der Verbindung mit 1,74 g (0,01 Mol) 2,4-Diisocyanatotoluol intensiv vermischt und bei 130 °C/2 h zu einem NCO-Gruppen-freien Kunststoff umgesetzt.

**Ansprüche**

1. Isocyanato-oxazolin-2-one der Formel

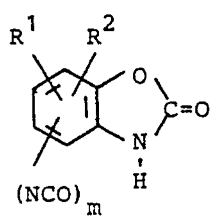

in welcher

$R^1$ und $R^2$ entweder für gleiche oder verschiedene Reste stehen und Wasserstoff, einen aliphatischen Kohlenwasserstoffrest mit 1 bis 4 Kohlenstoffatomen oder Chlor bedeuten oder gemeinsam für einen ankondensierten, gegebenenfalls mit einer Isocyanatgruppe substituierten Benzolring stehen oder wobei

$R^1$ die genannte Bedeutung hat und $R^2$ für einen Rest der Formel

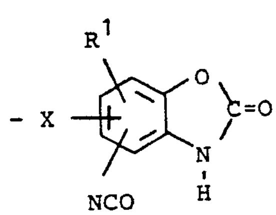

steht, wobei

X für eine Alkylen-Brücke steht und

m für 1 steht bzw. im Falle des Vorliegens eines Isocyanato-substituierten, ankondensierten aromatischen Rings auch für 0 stehen kann.

2. Verfahren zur Herstellung von unter dem Einfluß von Hitze und gegebenenfalls in Gegenwart von Kettenverlängerungs- bzw. Vernetzungsmitteln zu hochmolekularen, gegebenenfalls vernetzten Kunststoffen ausreagierenden, Oxazolin-2-on-Ringe aufweisenden Kunststoffvorläufern, dadurch gekennzeichnet, daß man mindestens zwei Hydroxylgruppen und/oder Aminogruppen aufweisende Verbindungen der als Reaktionspartner für organische Polyisocyanate aus der Polyurethanchemie bekannten Art mit Isocyanato-oxazolin-2-onen gemäß Anspruch 1 im Sinne einer Additionsreaktion, gegebenenfalls unter Mitverwendung von Oxazolin-2-on-freien Polyisocyanaten der aus der Polyurethanchemie bekannten Art ein- oder zweistufig, umsetzt, wobei man die Reaktionspartner in (i) einem Äquivalentverhältnis von Isocyanatgruppen des Isocyanatoxazolin-2-ons zu gegenüber Isocyanatgruppen reaktionsfähigen Gruppen des Kunststoffvorläufers von 3 : 1 bis 1 : 3 und (ii) einem Äquivalentverhältnis von Isocyanatgruppen der Oxazolin-2-on-freien Polyisocyanate in gemäß (i) gegebenenfalls vorliegenden überschüssigen, gegenüber Isocyanatgruppen reaktionsfähigen Gruppen von 0 : 1 bis 3 : 1 entsprechenden Mengen einsetzt.

3. Verfahren gemäß Ansprüchen 2, dadurch gekennzeichnet, daß man die Kunststoffvorläufer mit gegenüber Isocyanatgruppen reaktionsfähigen Gruppen in einem Äquivalentverhältnis von Isocyanatgruppen zu gegenüber Isocyanatgruppen reaktionsfähigen Gruppen von 1 : 0,95 bis 1 : 2,5 entsprechenden, Mengen einsetzt.

4. Verwendung der gemäß Ansprüchen 2 bis 3 erhältlichen Oxazolin-2-on-Ringe aufweisenden Kunststoffvorläufer zur Herstellung von hochmolekularen Kunststoffen, dadurch gekennzeichnet, daß man diese Kunststoffvorläufer, gegebenenfalls in Gegenwart von Verbindungen, die mindestens 2, mit Oxazolin-2-on-Ringen im Sinne einer Additions- oder Kondensationsreaktion reaktionsfähigen Gruppen enthalten, ausgewählt aus der Gruppe bestehend aus organischen Polyisocyanaten, organischen Polyaminen mit primären und/oder sekundären Aminogruppen und organischen Epoxiden, einer Hitzebehandlung im Bereich von 100 bis 200 °C unterwirft.

**Claims**

1. Isocyanato-oxazolin-2-ones of the formula

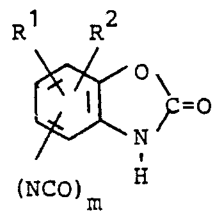

in which

$R^1$ and $R^2$ either represent identical or different radicals and denote hydrogen, an aliphatic hydrocarbon radical with 1 to 4 carbon atoms or chlorine, or together represent a condensed benzene ring optionally substituted by an isocyanate group, or wherein

$R^1$ has the above-mentioned meaning and $R^2$ represents a radical of the formula

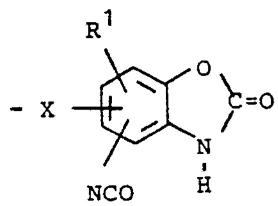

wherein

X represents an alkylene bridge and

m represents 1 or, where an isocyanato-substituted condensed aromatic ring is present, can also represent 0.

2. Process for the production of plastics precursors containing oxazolin-2-one rings which react fully under the influence of heat and optionally in the presence of chain-extending or cross-linking agents to form high molecular weight, optionally cross-linked plastics, characterised in that compounds of the type known from polyurethane chemistry as reactants for organic polyisocyanates and containing at least two hydroxyl groups and/or amino groups are reacted in one or two stages with isocyanato-oxazolin-2-ones according to Claim 1 in an addition reaction, optionally in the presence of oxazolin-2-one-free polyisocyanates of the type known in polyurethane chemistry, the reactants being used in quantities corresponding (i) to an equivalent ratio of isocyanate groups in the isocyanato-oxazolin-2-one to isocyanate-reactive groups in the plastics precursor of 3 : 1 to 1 : 3 and (ii) to an equivalent ratio of isocyanate groups in the oxazolin-2-one-free polyisocyanates to any excess isocyanate-reactive groups which may be present according to (i) of 0 : 1 to 3 : 1.

3. Process according to Claims 2, characterised in that the plastics precursors containing isocyanate-reactive groups are used in quantities corresponding to an equivalent ratio of isocyanate groups to isocyanate-reactive groups of 1 : 0.95 to 1 : 2.5.

4. Use of the plastics precursors containing oxazolin-2-one rings obtainable in accordance with Claims 2 to 3 for the production of high molecular weight plastics, characterised in that these plastics precursors are subjected to a heat treatment in the range of 100 to 200 °C, optionally in the presence of compounds which contain at least 2 groups capable of reacting with oxazolin-2-one rings in an addition or condensation reaction, these compounds being selected from the group consisting of organic polyisocyanates, organic polyamines containing primary and/or secondary amino groups and organic epoxides.

**Revendications**

1. Isocyanato-oxazoline-2-ones de formule

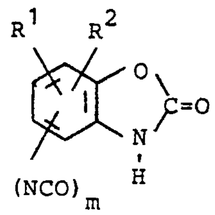

dans laquelle

$R^1$ et $R^2$ sont identiques ou différents et représentent chacun l'hydrogène ou le chlore ou un reste d'hydrocarbure aliphatique en $C_1$-$C_4$, ou bien $R^1$ et $R^2$ représentent ensemble un noyau benzénique condensé, éventuellement substitué par un groupe isocyanate, ou bien

$R^1$ a la signification mentionnée et $R^2$ représente un reste de formule

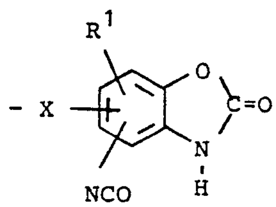

dans laquelle

X représente un pont alkylène et

m est égal à 1, ou bien, dans le cas de la présence d'un noyau condensé isocyanato-substitué, m peut aussi être égal à 0.

2. Procédé pour la fabrication de précurseurs de matières plastiques à noyaux oxazoline-2-one, réagissant complètement sous l'influence de la chaleur et éventuellement en présence d'agents d'allongement de chaînes ou d'agents réticulants en matières plastiques de hauts poids moléculaires, éventuellement réticulés, caractérisé en ce que l'on fait réagir des composés présentant au moins deux groupes hydroxyles ou groupes amino, du type connu dans la chimie des polyuréthannes comme partenaires de réaction pour les polyisocyanates organiques, avec des isocyanato-oxazoline-2-ones selon la revendication 1 au sens d'une réaction d'addition, en un ou deux stades, éventuellement avec utilisation simultanée de polyisocyanates exempts d'oxazoline-2-one, du type connu dans la chimie des polyuréthannes, en utilisant les partenaires de réaction en quantités correspondant à (i) un rapport d'équivalents des groupes isocyanates de l'isocyanato-oxazoline-2-one aux groupes du précurseur de matière plastique réactifs vis-à-vis des groupes isocyanate de 3 : 1 à 1 : 3 et à (ii) un rapport d'équivalents des groupes isocyanate des polyisocyanates exempts d'oxazoline-2-one aux groupes réactifs vis-à-vis des isocyanates éventuellement présents en excès selon (i) de 0 : 1 à 3 : 1.

3. Procédé selon la revendication 2, caractérisé en ce que l'on utilise les précurseurs de matières plastiques à groupes réactifs vis-à-vis des groupes isocyanate en quantités correspondant à un rapport d'équivalents des groupes isocyanate aux groupes réactifs vis-à-vis des isocyanates de 1 : 0,95 à 1 : 2,5.

4. Utilisation des précurseurs de matières plastiques à noyaux oxazoline-2-one, obtenus selon les revendications 2 et 3, pour la fabrication de matières plastiques de haut poids moléculaire, caractérisée en ce que l'on soumet ces précurseurs de matières plastiques à un traitement thermique dans l'intervalle de 100 à 200 °C, éventuellement en présence de composés qui contiennent au moins deux groupes réactifs avec les noyaux oxazoline-2-one au sens d'une réaction d'addition, qui sont choisis parmi les polyisocyanates organiques, les polyamines organiques à groupes amino primaires et/ou secondaires et les époxydes organiques.